# EUROPEAN PATENT SPECIFICATION

(11) **EP 3 829 698 B1**
(45) Date of publication and mention of the grant of the patent: **09.08.2023**
(21) Application number: 19844948.0
(22) Date of filing: 29.07.2019
(51) Int. Cl.: A61N 1/05, A61B 5/291, A61B 5/369

(54) **TRANSCRANIAL CURRENT LOOP STIMULATION DEVICE**
TRANSKRANIELLE STROMSCHLEIFENSTIMULATIONSVORRICHTUNG
DISPOSITIF DE STIMULATION TRANSCRÂNIENNE À BOUCLE DE COURANT

(30) Priority: 30.07.2018 US 201862711915 P
(43) Date of publication of application: 09.06.2021
(73) Proprietor: Epic Neuro, Inc., Fountain Valley, California 92708 (US)
(72) Inventor: PHILLIPS, James William, Fountain Valley, California 92708 (US)
(74) Representative: Clark, Jane Anne
(86) International application number: PCT/US2019/043884
(87) International publication number: WO 2020/028233

(56) References cited:
- WO-A1-2015/021075
- WO-A1-2016/030822
- US-A1- 2007 225 773
- US-A1- 2010 125 312
- US-A1- 2011 213 382
- US-A1- 2016 199 656
- US-A1- 2017 021 162
- US-B1- 9 278 208

## Description

### BACKGROUND OF THE INVENTION

Electric brain stimulation has been shown to be a potentially effective treatment for a number of brain disorders, including epilepsy, migraine, fibromyalgia, coma, major depression, stroke rehabilitation, and Parkinson's disease, and is also used in electrocorticography and Cortical Stimulation Mapping (CSM). Traditional Deep Brain Stimulation (DBS) is a neurosurgical procedure involving implantation of an Implantable Pulse Generator (IPG) placed under the skin near the collarbone. The IPG is battery powered and sends electrical pulses through a lead, which is routed under the skin to an area near the top of the patient's head, where it separates into two leads, each with its own set of electrodes near the tip. The leads proceed through the skull via two burr holes and are implanted so that the target region is between the two electrodes. Deep Brain Stimulation has been approved by FDA for treatment of essential tremor, Parkinson's disease, dystonia, obsessive-compulsive disorder, and epilepsy.

Traditional DBS systems suffer from disadvantages for the patient and the physician. The implantation procedure tends to be several hours in duration and involve a long recovery time. The tunneled leads cause significant pain and discomfort to the patient. The IPG requires a pocket incision that is prone to infection. The IPG itself is susceptible to "twiddle factor", in which the patient pushes the IPG around in their chest. The IPG may be visible and unsightly in the chest. The devices are very difficult to remove. The IPG must be replaced periodically due to battery drain. The IPG replacement issue may be avoided by having a rechargeable battery, but the other disadvantages remain a problem.

It would be advantageous to have a DBS system that did not involve an IPG implanted in the chest, with tunneled leads. Instead, one could create a modular IPG that is implanted in a space inside a burr hole in the patient's head with a lead that extends on or into the brain, and a subcutaneous electrode. If two modular IPGs are implanted near each other, the electric pulses from the two IPGs can be synchronized so that their pulses add, creating a current loop through one modular IPG, the brain target, the other modular IPG, then through the scalp back to the first modular IPG. Such a system would have several advantages over existing DBS technology, in that the IPGs can be smaller (since there are two of them sharing the load), the surgery is simpler with no tunneled leads and no pocket incision. Recovery time is shorter. The device is much more easily removable and is not visible to the patient. If the IPGs had a rechargeable battery, then no battery replacement would be required. A device according to the preamble of claim 1 is known from US2010/125312.

### BRIEF SUMMARY OF THE INVENTION

In broad terms, the present invention, which is defined in claim 1, provides electrical stimulation to a brain of a person, the device comprising a first module and a second module, each module comprising: (a) case; and (b) a seal; and (c) a battery; and (d) a lead with at least one subcranial electrode; and (e) a subcutaneous electrode; and (f) control circuitry implementing control logic, wherein the battery and the control circuitry is contained inside the case and configured to generate electric pulses at a pulse frequency, a pulse amplitude, a pulse width, and a duty cycle, the case is configured to occupy a space within a burr hole or craniotomy in a skull, the lead is configured to extend near or within the brain, the subcutaneous electrode is configured to lie above the skull and under the scalp, and the seal is configured to fill a gap between the case and an edge of the burr hole or craniotomy to minimize electrical current between at least one subcranial electrode and the subcutaneous electrode.

The two modules may be implanted through burr holes or cranitomies, with electric pulses from each module synchronized to provide additive stimulation. In one aspect, the modules are each implanted through burr holes or craniotomies in a skull, and the control circuitry with programmed logic of each module is configured to synchronize the electric pulses between the modules so that electric current forms a loop, allowing current to flow interior and exterior to the skull and through at least one stimulation target.

The battery may be rechargeable, which would obviate the need for battery or module changes, involving surgery. The battery may be chosen to provide enough power to ensure stimulation for many days, weeks, or months, allowing the person to recharge the battery of each module during regular visits to the clinician. In one aspect, the battery is rechargeable, each module further comprising a coil and a recharging circuit, configured to recharge the battery through magnetic inductive coupling with an external magnetic field generator.

The first and second modules may be kept separate, forming a current loop that includes the brain and the scalp. However, a conductive wire may be used to connect the subcutaneous electrodes of the modules together, which would remove the impedance contribution from the scalp. This could reduce the required voltage of the modules and could improve longevity. However, it would involve a tunneled wire, which increases the complexity of the surgery and potential side effects. In one aspect, the device further comprises a conductive wire that connects the subcutaneous electrodes of the first module and the second module together. In this case, the subcutaneous electrodes do not need to touch the scalp tissue directly, allowing for one continuous shielded current path from one module to the other underneath the scalp.

The lead of each module includes one or more electrodes. It is important to ensure effective stimulation that the region of the brain responsible for the disorder be accurately targeted. In some instances, surgeons do not know the optimal stimulation location. Therefore, it is often advantageous to include a plurality of electrodes in each lead, and allow the clinician to specify one or more active electrodes in each lead in order to effectively target a selected region and to bring about an improvement in psychological or physiological health of the brain. In one aspect, the control circuitry with programmed logic is further configured to select one or more of a plurality of subcranial electrodes to be active.

When the modules of the device are implanted, it may be necessary to inform the person, the clinician, or the caregiver of information related to the device. In one aspect, the first module further comprises a speaker configured to provide audio information to the person, a clinician, or a caregiver. The type of information may include device status and programming parameters. In one aspect, the audio information comprises battery level, pulse frequency, pulse amplitude, pulse width, duty cycle, active electrode of the first module, active electrode of the second module, charging status, and therapy status. The audio information may use a speaker or piezoelectric crystal. The format of the audio signal may take a variety of forms. In one aspect, the audio information is generated as a series of beeps, ticks, or tones, which can be decoded.

For information to pass from a programmer to the device or from the device to the programmer, it may be necessary for the device to include an antenna. In one aspect, each module further comprises an antenna, wherein the antenna may receive data from or transmit data to an external programmer. The programmer may be used, for example, to program treatment parameters, initiate treatment, terminate treatment, synchronize stimulation pulses, or request information from the device. In one aspect, the control logic of each module is configured to program treatment parameters of the module based on commands from the external programmer, the treatment parameters comprising active electrode selection, pulse frequency, pulse amplitude, pulse width, duty cycle, therapy initiation and therapy cessation. In one aspect, the control logic of each module is configured to provide information to the external programmer, the information comprising battery level, pulse frequency, pulse amplitude, pulse width, duty cycle, active electrode of the first module, active electrode of the second module, charge status, and therapy status.

In addition to stimulation, it may be necessary to record the electroencephalogram (EEG) of the person's brain. The EEG data may be used by the control circuitry with programmed logic to adjust stimulation parameters. It may also be transmitted to the external programmer for analysis or diagnosis. In one aspect, the device further comprises an EEG amplifier and analog-to-digital converter, allowing the device to record an EEG of the person. To store the EEG for use or transmission, storage memory may be incorporated into the device. In one aspect, the device further comprises memory configured to store the EEG of the person, and a transmitting antenna, wherein the control circuitry with programmed logic transmits the stored EEG through the transmitting antenna.

It may be necessary to initiate activities in the device without the use of an external programmer. One means to do this is to use an external permanent magnet that activates a magnetic switch in the device when the permanent magnet is brought close to the scalp near a module. For example, an EEG recording may be initiated, paused, or ceased, or stimulation may be started or stopped using the external permanent magnet. In one aspect, the device further comprises a magnetic switch, wherein starting or stopping of an EEG recording is controlled by the person placing a permanent magnet close to the magnetic switch, or by the person moving the permanent magnet away from the magnetic switch. In one aspect, the device further comprises a magnetic switch, wherein starting or stopping of stimulation is controlled by the person placing a permanent magnet close to the magnetic switch, or by the person moving the permanent magnet away from the magnetic switch. Such a permanent magnet may be carried in the person's pocket for on-demand use.

Positioning of the electrodes of the device to target a specific region of the brain of the person allows for the treatment of mental disorders and improving a psychological or physiological condition of the person. In one aspect, the lead of each module is configured to be implanted so that one or more of the subcranial electrodes are in or near an anterior nucleus of a thalamus, which allows for the treatment of epilepsy. In one aspect, the lead of each module is configured to be implanted so that one or more of the subcranial electrodes are in or near a nucleus accumbens, which allows for treatment of addiction, such as from alcohol or opioids. In one aspect, the lead of each module is configured to be implanted so that one or more of the subcranial electrodes are in or near a subthalamic nucleus or globus pallidus interna, which allows for the treatment of Parkinson's disease and other movement disorders. In one aspect, the lead of each module is configured to be implanted so that one or more of the subcranial electrodes are in or near a hippocampus, which allows for treatment of depression or epilepsy. In one aspect, the lead of each module is configured to be implanted so that one or more of the subcranial electrodes are in or near a subcallosal cingulate, which allows for treatment of depression. In one aspect, the lead of each module is configured to be implanted so that one or more of the subcranial electrodes are in or near an anterior nucleus of a pituitary gland, which may stimulate hormone production. In one aspect, the lead of each module is configured to be implanted so that one or more of the subcranial electrodes are in or near a mesencephalic reticular formation or non-specific thalamic nucleus, which allows for the treatment of persistent vegetative state, or coma. In one aspect, the lead of each module is configured to be implanted so that one or more of the subcranial electrodes are in or near a cortex. Stimulation of cortical targets may help in a variety of disorders, including depression, anxiety, post traumatic stress disorder, autism, traumatic brain injury, stroke.

### BRIEF DESCRIPTION OF THE DRAWINGS

FIG. 1 is a drawing of one aspect of the device, which shows a case, subcutaneous electrode, and coil with a lead extending from the case, and four selectable electrodes near the distal end of the lead.
FIG. 2 is a drawing of one aspect of the device, showing the brain with two modules of the device implanted, in which the case of the IPG is flush with the skull and the subcutaneous electrode and coil are underneath the scalp. The leads of both modules extend into the brain, with the lead length and electrode placement chosen to affect a target region in the brain.
FIG. 3 is an electric schematic drawing of one aspect of the device, showing the current loop for two modules of the device, in which the electrical pulse generation of the two modules is synchronized. The drawing shows all impedance elements of the current path as well.
FIG. 4 is a drawing which shows an example of a charger, which is contained in a headband worn by the patient. The charger could also serve as a programmer as well.

### DETAILED DESCRIPTION OF THE INVENTION

While certain embodiments have been provided and described herein, it will be readily apparent to those skilled in the art that such embodiments are provided by way of example only. It should be understood that various alternatives to the embodiments described herein may be employed and are part of the invention described herein.

Provided herein is a device whereby electrical current stimulation is applied to a brain of a person through the implantation of one or more modules through a burr hole or craniotomy in a skull of the person. Each module can be divided into two primary units: The Implantable Pulse Generator (IPG) and the lead. The IPG is contained inside or on a case and is placed into a burr hole or craniotomy that approximately matches the size of the IPG case and positioned so that the top of the IPG is approximately flush with the outer surface of the skull. The IPG comprises a battery, control circuitry with programmed logic, and a subcutaneous electrode. The primary function of the IPG is to generate electrical pulses of a specified frequency, amplitude, pulse width, and duty cycle, in order to provide the current stimulation of a target in or on the brain. The lead extends from the IPG and comprises one or more subcranial electrodes at or near the distal end of the lead. The length of the lead and the number and position of subcranial electrodes along the lead are chosen so that one or more of the electrodes will be on or near a target region in the brain of the person. The control circuitry with programmed logic in the IPG selects one or more electrodes to be active, and the remaining electrodes to be inactive, providing no path to current flow. A seal may be positioned to fill the gap between the edge of the IPG and the edge of the burr hole or craniotomy. The seal is intended to minimize or prevent electric current flow between the subcutaneous electrode and the active subcranial electrode.

If a single module is implanted in the person, then a second burr hole or craniotomy may be cut through the skull in order to provide a return path for current flow between the subcutaneous electrode and the active subcranial electrode. Alternately, a purposeful gap may be left between the IPG and the edge of the burr hole or craniotomy to allow a return path for the current. When an electrical current pulse is generated in the IPG, a current loop is formed that goes through the lead to one or more active electrodes, into the brain through one or more target regions, through the second burr hole or craniotomy, through the scalp, through the subcutaneous electrode, and back to the IPG.

Alternately, the control circuitry with programmed logic in the IPG could select two electrodes in the lead to be active and provide stimulation between the two active electrodes, and not involve the subcutaneous electrode. In this case, a second burr hole or seal would not be necessary.

A first module and a second module may be implanted in the person, each with its own burr hole or craniotomy. In this case, the electric current pulses from the IPGs in the two modules can be synchronized so that the resulting voltages are additive, forming a current loop that goes through the lead of the first module to one or more active electrodes of the first module's lead, into the brain through one or more target regions, through one or more active electrodes of the second module's lead, through the second module's IPG and subcutaneous electrode, through the scalp, through the subcutaneous electrode of the first module, and back to the first module's IPG.

If two modules are implanted, then alternately both modules could occupy the same burr hole or craniotomy. If this is the case, then the subcutaneous electrodes of each module could be connected together, eliminating any substantial electrical current flow through the scalp. If the two modules are implanted in separate burr holes or craniotomies, then the electrodes may be connected by a conductive wire, which also eliminates any substantial electrical current flow through the scalp. In this case, the subcutaneous electrodes do not need to touch the scalp tissue directly, allowing for one continuous shielded current path from one module to the other underneath the scalp. This has the advantage of lowering the overall impedance through the current path and making it less likely that electrical current through the scalp will be sensed by the person. However, the conductive wire will need to be tunneled from one module to the next, involving additional surgery and potential for complications.

The length of the lead for each module and position of electrodes is configured in order to best provide electrical current stimulation to a target region. In addition, stimulation parameters comprising pulse frequency, pulse amplitude, pulse width, and duty cycle can be chosen based on the indication. For example, stimulation of the Anterior Nucleus of the Thalamus with stimulation set to 5V, 145 pulses per second, 90 microsecond pulse width, and a duty cycle of 1 minute on, 4 minutes off, has been shown to be an effective treatment for epilepsy. In another example, stimulation of the nucleus accumbens with stimulation set to 3.5V, 130 pulses per second, and 90 microsecond pulse width has shown benefit in patients with addition, such as with opioids or alcohol. Many studies have been conducted using electrical stimulation to treat Parkinson's. In general, stimulation of the subthalamic nucleus or globus pallidus internus using stimulation set to 2.4 to 4.4V, frequency in the range of 143 to 173 pulses per second, and pulse widths in the range 67 to 138 microseconds have been found to effectively control motor symptoms. Stimulation of the subgenual cingulate cortex, the subcallosal cingulate, the ventral capsule or ventral striatum, nucleus accumbens, or medial forebrain bundle at a variety of treatment parameters has shown to be effective for treating major depressive disorder (MDD). Stimulation of the ventral capsule or ventral striatum, the subthalamic nucleus, or inferior thalamic peduncle using various treatment parameters have shown efficacy in treating Obsessive Compulsive Disorder (OCD). Stimulation of the mesencephalic reticular formation or non-specific thalamic nucleus has been shown to improve the symptoms in patients who are in a persistent vegetative state, or coma.

In one aspect, the device allows for recharging of the batteries of each module. The IPG can further comprise a coil, which allows for inductive coupling with an external magnetic field source, and a charging circuit can recharge the battery in the IPG. The recharging can take place at regular intervals. For example, if a 5V, 145 pulses per second, 90 microsecond pulse width, and a duty cycle of 1 minute on 4 minutes off, stimulation is provided by each module with a 1,000 ohm impedance for each module, and the battery has a capacity of 120 milliamp hours, stimulation can be provided for over 300 days before the battery will be drained. In this example, recharging once every 3-6 months will serve to keep the device functional. An external magnetic field generator can be kept at the clinician's office, and the patient can recharge the device during regular office visits.

Other methods of recharging the batteries may be used. In one example, ultrasonic charging may be performed using a piezoelectric crystal in the IPG. Another example is solar recharging using solar cells in the IPG. In this case, the skull is ideal for IPG location, since the scalp is relatively thin and generally uncovered, except for hair. The person recharges the device by exposing their head to light. Another example is recharging due to natural body movement. Another example is recharging due to the temperature differential between the subcutaneous and subcranial locations in the IPG.

It is important that the IPG be implanted in the burr hole or craniotomy so that it is flush, and that the lead be directed toward the desired location to provide proper stimulation to the target. Therefore, in one aspect, the lead is bendable, either at the end proximal to the IPG, or along the lead itself.

In order to control the modules, an external programmer may be necessary, which writes data to each module. Data may comprise treatment parameters such as pulse amplitude, pulse frequency, pulse width, duty cycle, active electrode selection, therapy initiation, and therapy cessation. In one aspect, the IPGs further comprise an antenna and receiver, which receive data from the programmer and pass that to the control circuitry with programmed logic. Examples include a Bluetooth module and wi-fi module, or magnetic coupling. It is also possible to use the same coil that is used for recharging the battery. In one aspect, frequency modulation is used to pass data to the IPG. In one aspect, amplitude modulation is used to pass data to the IPG.

Other means are also possible for the programmer to pass data to the IPG. For example, the IPG could incorporate an amplifier and analog to digital converter, and the control circuitry with programmed logic can be configured to recognize electrical signals detected between two or more electrodes. The programmer could use electrodes on the skin of the person and generate low level pulses that cannot be felt, but that can be detected by the control circuitry with programmed logic in the IPG through the receiving electrodes.

Since multiple modules may be implanted in the person, it may be necessary to program IPGs individually or collectively. To do this, in one aspect the control circuitry with programmed logic of each IPG has a unique identifier, and the programmer specifies the module or modules that a particular portion of data or command is directed towards.

It may also be necessary for modules to send data to the programmer. Such data may comprise battery level, pulse frequency, pulse amplitude, pulse width, duty cycle, charge status, therapy delivery status, active electrode or active electrodes. This data can be transmitted via a dedicated antenna. Examples include a Bluetooth module and wi-fi module, or magnetic coupling. It is also possible to use the same coil that is used for recharging the battery to pass data back to the programmer.

It may also be necessary to pass information to the person, directly without the use of a programmer. For example, if the battery level drops below a threshold, the IPG could generate a beep, click, or tone through a speaker to inform the person. Another means by which the IPG can pass information directly to the person is through the use of a vibrating unit. Even though the devices are implanted, the person should be able to sense a beep, click, or tone, due to the generally high sound conductivity of the body. Also, vibration should be able to be felt, due to the proximity of the IPG to the inner ear.

When charging, it may be necessary to inform the person that the battery is at or near a full charge. This could be accomplished, for example, by generating a beep, click, tone, or vibration. It would be possible for the IPG to use beeps, clicks, tones of varying frequencies, or varying vibration to encode information to the person. For example, a low tone followed by a high tone may indicate that the battery is at or near a full charge, whereas a high tone followed by a low tone may indicate that the battery is nearly depleted. The IPG could use beeps, clicks, tones, or vibration to encode more complex information. For example, to specify which electrode is active, the IPG could emit two high tones, followed by clicks or beeps to indicate which electrodes in the module are active and which are inactive.

In one aspect of the device, an EEG is recorded by the IPG. In this case, each module would further comprise memory to store the EEG. The module may use a dedicated antenna or the coil itself in order to transmit the stored EEG data to the programmer. In this case, the programmer may issue a command for the module to transmit the stored EEG. Once that command is given, the control circuitry with programmed logic would transmit the EEG data, for example, in a serial fashion, to the external programmer. The programmer may then issue a command for the module to clear memory in order to record additional EEG. An EEG recording may begin as a result of a command from the programmer, or it may begin at a predetermined interval, or as the result of a specific event. One way that EEG recording could be initiated is by the person or caregiver holding a permanent magnet near the module. In this case, the module would further comprise a magnetic switch. When the magnetic switch is tripped, an EEG recording may begin. The recording could end after a specified duration, or when a command is given by the programmer, or when a permanent magnet is brought close to the module or moved farther away from the module. A magnetic switch may also be used by the person to suspend or initiate therapy, independent of recording EEG.

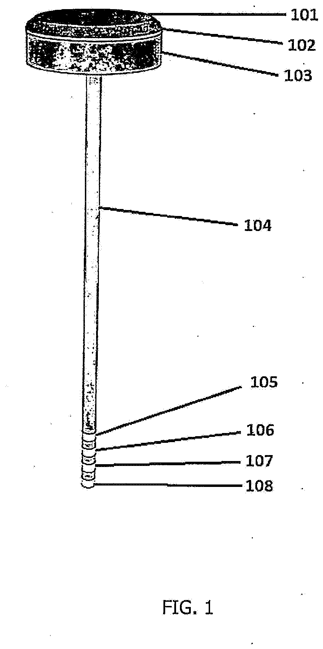

With reference to FIG. 1, a drawing is shown which presents an aspect of the device. The IPG comprises a case (103), which comprises a battery and control circuitry with programmed logic. If EEG is implemented, then the IPG further comprises an EEG amplifier and an analog to digital converter. The IPG further comprises a subcutaneous electrode (102) and a coil (101), used to recharge the battery. A lead (104) extends from the IPG. Four electrodes (105, 106, 107, 108) are located near the distal end of the lead. The length of the lead is chosen so as to ensure that one or more of the electrodes is on or near a region of the brain targeted for stimulation. The IPG is implanted in a burr hole in the skull, preferably so that the top and bottom sides of the IPG are approximately flush with the top and bottom of the skull, respectively. Although this aspect shows four electrodes, fewer electrodes may be used in order to minimize complexity and cost, or more electrodes may be used in order to provide more options. If EEG is being recorded by the device, any two electrodes may be selected for EEG recording, and the EEG recording electrodes may or may not be the same as the electrodes used for stimulation.

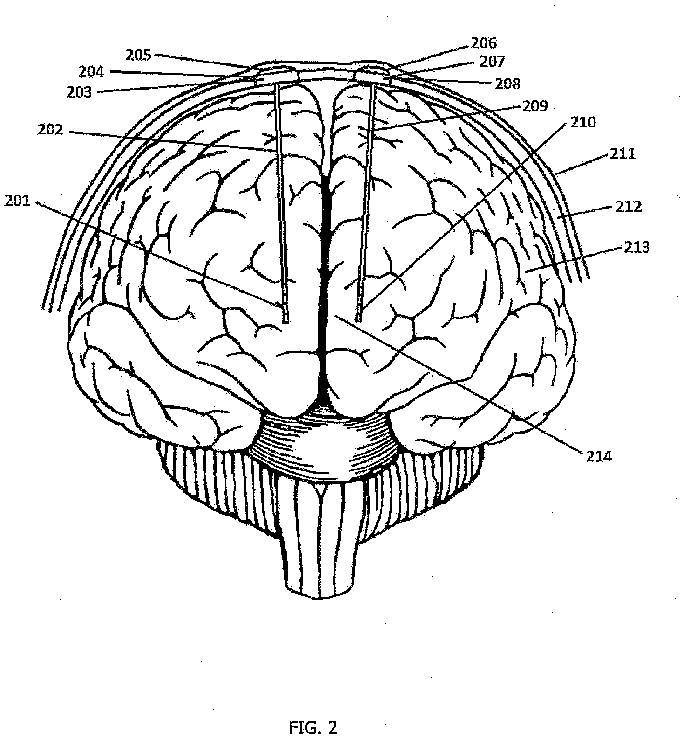

With reference to FIG. 2, a drawing is shown which presents a typical application of the device, in which two modules are implanted. The IPGs of the two modules are implanted so that they are approximately flush with the skull (212). The IPGs comprise a case (203, 208), subcutaneous electrode (204, 207), and a coil (205, 206). The coil is under the scalp (211) and may result in a slight bump in the scalp surface, but it should not be noticeable. Leads (202, 209) extend from each IPG into the brain (213) and comprise a number of electrodes. Active electrodes (201, 210) are selected in each lead (in order to provide stimulation to a targeted region (214) in the brain.

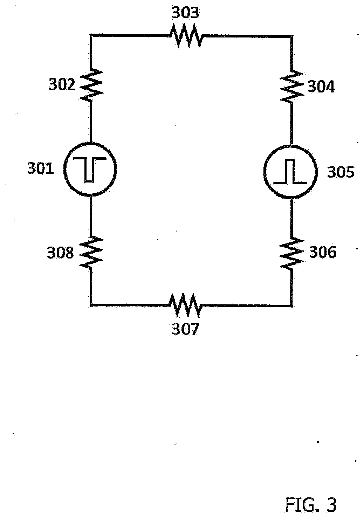

With reference to FIG. 3, an electrical schematic is shown, which models the synchronized pulses between two modules of the device. The first module (301) generates a negative pulse. For example, the pulse amplitude is -5V, pulse width is 90 microseconds. At approximately the same instant, the second module (305) generates a positive pulse. For example, the pulse amplitude may be 5V and pulse width is 90 microseconds. The voltages of the two devices sum together to create a 10V, 90 microsecond pulse. This forms a current loop through the interface between the first module (301), the active electrode of the first module and the brain tissue (308), the brain tissue that includes the target region (307), the interface between the active electrode of the second module and the brain tissue (306), the second module (305), the interface between the subcutaneous electrode of the second module and the scalp (308), the scalp between the two subcutaneous electrodes (303), the interface between the subcutaneous electrode of the first module and the scalp (302), and back to the first module (301). In general, the impedance between an electrode and surrounding brain or scalp tissue is approximately 500 ohms. The impedance of the brain in the target region and the scalp are very small. Therefore, the overall impedance of the system is approximately 2,000 ohms. With a 10V synchronized pulse, the current flow will be approximately 5 milliamps. If the electrodes are connected by a conductive wire, then the impedance contribution from the subcutaneous electrodes and scalp (302, 303, 304) would effectively been eliminated, which would approximately double the current flow through the target region in the brain.

In order to synchronize pulses between modules, it may be necessary for the two to communicate. This could be done, for example, using a master-slave relationship. The first module could generate a very short and low amplitude pulse at a defined time before the therapy pulse is delivered. This would be detected by the second module, either through the coil or through the electrodes to an amplifier and analog to digital converter, and the second module would then be able to generate a pulse in sync with the first module. Alternately, the second module could monitor when the first module begins its therapy pulse and adjust its timing accordingly so that all pulses are aligned. Alternately, timing could be established through the external programmer, where the devices are synchronized and each module comprises a clock or timing mechanism that is of sufficient accuracy that the two modules stay in sync. In this case, modules can be re-synced using the external programmer during office visits.

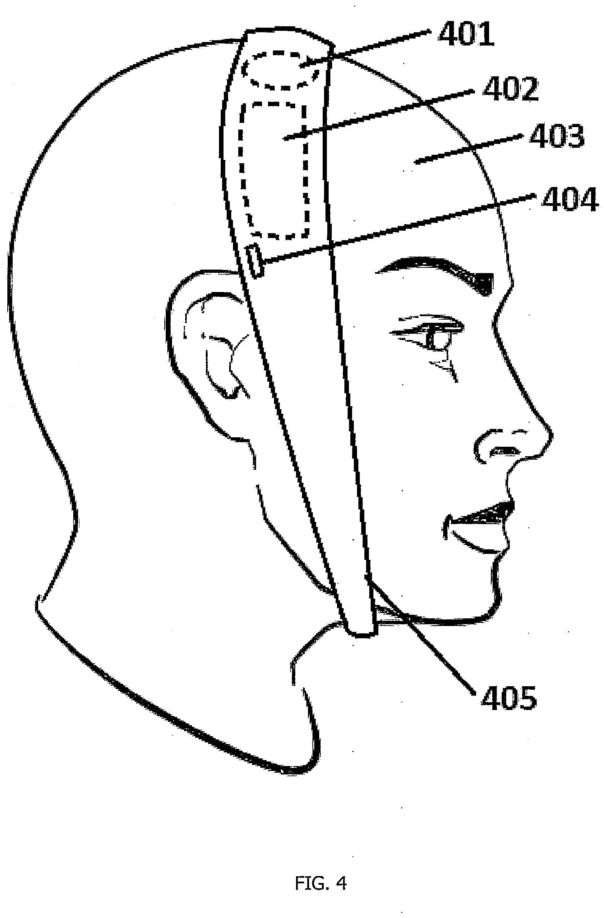

With reference to FIG. 4, a drawing is shown which presents a typical charger. A headband (405) is worn on the head (403) of the person so that the charging coil (401) is positioned over or near the first implanted module. Another charging coil is also positioned over or near the second implanted module. In this example, a battery 402 is contained in the headband, which will allow charging to occur without the person being tied to an external power supply. A charging port (403) may be included to allow recharging of the charger's battery. It is not required that the charger be a headband. It could instead be a wearable headset, a cap, or any means by which the charging coils are reliably positioned over or near the implanted modules. The charger does not even need to be worn. Instead, the charging coils could be part of a mount that is positioned near the implanted modules.

## Claims

1. A system comprising a first device (201-205) and a second device (206-210), the system being configured for electrical stimulation of a brain of a person, the first device and the second device each comprising:
a case (103) configured to occupy a space within a burr hole or craniotomy in a skull;
a subcutaneous electrode (102); a lead (104) attached to a lower surface of the case and configured to be implanted through the burr hole or craniotomy in the person's skull;
at least one subcranial electrode (105, 106, 107, 108) disposed on a lower end of the lead so that said at least one subcranial electrode will be positioned at a target location in the patient's brain when the lead is implanted through the burr hole or craniotomy;
a battery within the case; and
control circuitry internal to the case configured to receive current from the battery and generate electric pulses at a pulse frequency, a pulse amplitude, a pulse width, and a duty cycle, selected to stimulate the patient's brain; and
wherein the control circuitry of the first device and the second device are configured to synchronize the electric pulses between the first device and the second device so that electric current forms a loop, allowing current to flow interior and exterior to the skull and through at least one stimulation target, **characterised in that** said subcutaneous electrode is on the case configured to lie above the skull and under the scalp.

2. The system of Claim 1, wherein the battery in the first device and in the second device is rechargeable, each battery further comprising a coil and a recharging circuit, configured to recharge the battery through magnetic inductive coupling with an external magnetic field generator.

3. The system of Claim 1 or 2, wherein the control circuitry of the first device and the second device is further configured to select one or more of a plurality of subcranial electrodes to be active.

4. The system of any of Claims 1 to 3, wherein the first device and the second device each further comprise an antenna, wherein each antenna may receive data from or transmit data to an external programmer, optionally wherein the control circuitry of the first device and the second device is further configured to program treatment parameters of each respective first device or second device based on commands from the external programmer, the treatment parameters selected from a group consisting of active electrode selection, pulse frequency, pulse amplitude, pulse width, duty cycle, therapy initiation and therapy cessation.

5. The system of Claim 1, wherein the control circuitry of each respective first device and second device further comprises an EEG amplifier and analog-to-digital converter, thereby enabling the first device and the second device to record an EEG of the patient.

6. The system of any of Claims 1 to 5, wherein each of the first device and the second device further comprises memory configured to store the EEG of the person (403), and a transmitting antenna, wherein the control circuitry of each respective first device and second device is configured to transmit the stored EEG through its respective transmitting antenna.

7. The system of any of Claims 1 to 6, further comprising a magnetic switch, wherein starting or stopping of an EEG recording is controlled by the person placing a permanent magnet close to the magnetic switch, or by the person moving the permanent magnet away from the magnetic switch.

8. The system of any of Claims 1 to 6, wherein each of the first device and the second device further comprises a magnetic switch, wherein starting or stopping of stimulation is controlled by the person placing a permanent magnet close to the magnetic switch, or by the person moving the permanent magnet away from the magnetic switch.

9. The system of any of Claims 1 to 8, wherein the lead of each respective first device and second device is configured to be implanted so that one or more of the subcranial electrodes are in or near an anterior nucleus of a thalamus, in or near a nucleus accumbens, in or near a subthalamic nucleus or globus pallidus interna, in or near a hippocampus, in or near a subcallosal cingulate, in or near an anterior nucleus of a pituitary gland, in or near a mesencephalic reticular formation or non-specific thalamic nucleus, or in or near a cortex of the patient.

10. The system of Claim 1, further comprising a conductive wire configured to connect the subcutaneous electrodes of the first device and the second device together.

11. The system of Claim 1, wherein the first device further comprises a speaker configured to provide audio information to the person, a clinician, or a caregiver.

12. The system of Claim 11, wherein the audio information is generated as a series of beeps, ticks, or tones, which can be decoded.

## Patentansprüche

1. Ein System, das eine erste Vorrichtung (201-205) und eine zweite Vorrichtung (206-210) beinhaltet, wobei das System zur elektrischen Stimulierung eines Gehirns einer Person ausgelegt ist, wobei die erste Vorrichtung und die zweite Vorrichtung jeweils Folgendes beinhalten:
ein Gehäuse (103), das dazu ausgelegt ist, einen Raum innerhalb eines Bohrlochs oder einer Kraniotomie in einem Schädel einzunehmen;
eine subkutane Elektrode (102);
eine Leitung (104), die an einer unteren Oberfläche des Gehäuses befestigt ist und dazu ausgelegt ist, durch das Bohrloch oder die Kraniotomie im Schädel der Person implantiert zu werden;
mindestens eine subkranielle Elektrode (105, 106, 107, 108), die an einem unteren Ende der Leitung angeordnet ist, sodass die mindestens eine subkranielle Elektrode an einem Zielort im Gehirn des Patienten positioniert sein wird, wenn die Leitung durch das Bohrloch oder die Kraniotomie implantiert wird;
eine Batterie innerhalb des Gehäuses; und
innen im Gehäuse befindliche Steuerschaltungen, die dazu ausgelegt sind, Strom von der Batterie zu erhalten und elektrische Impulse mit einer Impulsfrequenz, einer Impulsamplitude, einer Impulsbreite und einem Arbeitszyklus zu erzeugen, die ausgewählt werden, um das Gehirn des Patienten zu stimulieren; und
wobei die Steuerschaltungen der ersten Vorrichtung und der zweiten Vorrichtung dazu ausgelegt sind, die elektrischen Impulse zwischen der ersten Vorrichtung und der zweiten Vorrichtung so zu synchronisieren, dass der elektrische Strom eine Schleife bildet, wodurch der Strom innerhalb und außerhalb des Schädels und durch mindestens ein Stimulationsziel fließen kann, **dadurch gekennzeichnet, dass** die subkutane Elektrode sich auf dem Gehäuse befindet, das dazu ausgelegt ist, über dem Schädel und unter der Kopfhaut zu liegen.

2. System nach Anspruch 1, wobei die Batterie in der ersten Vorrichtung und in der zweiten Vorrichtung aufladbar ist, wobei jede Batterie ferner eine Spule und eine Ladeschaltung beinhaltet, die zum Wiederaufladen der Batterie durch magnetische Induktionskopplung mit einem externen Magnetfeldgenerator ausgelegt ist.

3. System nach Anspruch 1 oder 2, wobei die Steuerschaltungen der ersten Vorrichtung und der zweiten Vorrichtung ferner dazu ausgelegt sind, eine oder mehrere von einer Vielzahl subkranieller Elektroden dazu auszuwählen, aktiv zu sein.

4. System nach einem der Ansprüche 1 bis 3, wobei die erste Vorrichtung und die zweite Vorrichtung jeweils ferner eine Antenne beinhalten, wobei jede Antenne Daten von einem externen Programmiergerät erhalten kann oder Daten an dieses übermitteln kann, wobei die Steuerschaltungen der ersten Vorrichtung und der zweiten Vorrichtung optional ferner dazu ausgelegt sind, Behandlungsparameter von jeder jeweiligen ersten Vorrichtung oder zweiten Vorrichtung auf der Grundlage von Befehlen von dem externen Programmiergerät zu programmieren, wobei die Behandlungsparameter aus einer Gruppe ausgewählt sind, die aus Auswahl der aktiven Elektroden, Impulsfrequenz, Impulsamplitude, Impulsbreite, Arbeitszyklus, Einleitung der Therapie und Beenden der Therapie besteht.

5. System nach Anspruch 1, wobei die Steuerschaltungen jeder jeweiligen ersten Vorrichtung und zweiten Vorrichtung ferner einen EEG-Verstärker und Analog-Digital-Wandler beinhalten, wodurch ermöglicht wird, dass die erste Vorrichtung und die zweite Vorrichtung ein EEG des Patienten aufzeichnen.

6. System nach einem der Ansprüche 1 bis 5, wobei jede von der ersten Vorrichtung und der zweiten Vorrichtung ferner einen Speicher, der dazu ausgelegt ist, das EEG der Person (403) zu speichern, und eine Sendeantenne beinhaltet, wobei die Steuerschaltungen jeder jeweiligen ersten Vorrichtung und zweiten Vorrichtung dazu ausgelegt sind, das gespeicherte EEG durch ihre jeweilige Sendeantenne zu übermitteln.

7. System nach einem der Ansprüche 1 bis 6, das ferner einen Magnetschalter beinhaltet, wobei das Starten oder Stoppen einer EEG-Aufzeichnung dadurch gesteuert wird, dass die Person einen Dauermagnet in der Nähe des Magnetschalters platziert oder dass die Person den Dauermagnet von dem Magnetschalter entfernt.

8. System nach einem der Ansprüche 1 bis 6, wobei jede von der ersten Vorrichtung und der zweiten Vorrichtung ferner einen Magnetschalter beinhaltet, wobei das Starten oder Stoppen der Stimulation dadurch gesteuert wird, dass die Person einen Dauermagnet in der Nähe des Magnetschalters platziert oder dass die Person den Dauermagnet von dem Magnetschalter entfernt.

9. System nach einem der Ansprüche 1 bis 8, wobei die Leitung jeder jeweiligen ersten Vorrichtung und zweiten Vorrichtung dazu ausgelegt ist, so implantiert zu werden, dass eine oder mehrere der subkraniellen Elektroden sich in einem oder in der Nähe eines Nucleus anterior des Thalamus, in einem oder in der Nähe eines Nucleus accumbens, in einem oder in der Nähe eines Nucleus subthalamicus oder Globus pallidus interna, in einem oder in der Nähe eines Hippocampus, in einem oder in der Nähe eines Cingulum subcallosum, in einem oder in der Nähe eines Nucleus anterior der Hypophyse, in einem oder in der Nähe einer Formatio reticularis des Mesencephalons oder eines unspezifischen Nucleus thalami oder in einem oder in der Nähe eines Kortex des Patienten befinden.

10. System nach Anspruch 1, das ferner einen leitenden Draht beinhaltet, der dazu ausgelegt ist, die subkutanen Elektroden der ersten Vorrichtung und der zweiten Vorrichtung miteinander zu verbinden.

11. System nach Anspruch 1, wobei die erste Vorrichtung ferner einen Lautsprecher beinhaltet, der dazu ausgelegt ist, der Person, einem Klinikarzt oder Betreuer Audioinformationen bereitzustellen.

12. System nach Anspruch 11, wobei die Audioinformationen als eine Reihe von Piep-, Tick- oder Klangtönen, die entschlüsselt werden können, erzeugt werden.

## Revendications

1. Système comprenant un premier dispositif (201-205) et un deuxième dispositif (206-210), le système étant configuré pour la stimulation électrique du cerveau d'une personne, le premier dispositif et le deuxième dispositif comprenant chacun :
un boîtier (103) configuré pour occuper un espace à l'intérieur d'un trou de trépan ou d'une craniotomie dans un crâne ;
une électrode sous-cutanée (102) ;
un fil (104) fixé à une surface inférieure du boîtier et configuré pour être implanté par le trou de trépan ou la craniotomie dans le crâne de la personne ;
au moins une électrode sous-crânienne (105, 106, 107, 108) disposée à une extrémité inférieure du fil de telle sorte que ladite au moins une électrode sous-crânienne soit positionnée à un emplacement cible dans le cerveau du patient lorsque le fil est implanté par le trou de trépan ou la craniotomie ;
une batterie à l'intérieur du boîtier ; et
une circuiterie de commande à l'intérieur du boîtier configurée pour recevoir un courant en provenance de la batterie et générer des impulsions électriques à une fréquence d'impulsion, une amplitude d'impulsion, une largeur d'impulsion et selon un cycle de service, sélectionnés pour stimuler le cerveau du patient ; et
dans lequel la circuiterie de commande du premier dispositif et du deuxième dispositif est configurée pour synchroniser les impulsions électriques entre le premier dispositif et le deuxième dispositif de telle sorte que le courant électrique forme une boucle, en permettant au courant de circuler à l'intérieur et à l'extérieur du crâne et à travers au moins une cible de stimulation, **caractérisé en ce que** ladite électrode sous-cutanée est présente sur le boîtier configuré pour se trouver au-dessus du crâne et sous le cuir chevelu.

2. Système selon la revendication 1, dans lequel la batterie dans le premier dispositif et dans le deuxième dispositif est rechargeable, chaque batterie comprenant en outre une bobine et un circuit de recharge, configurés pour recharger la batterie par couplage magnétique inductif avec un générateur de champ magnétique externe.

3. Système selon la revendication 1 ou 2, dans lequel la circuiterie de commande du premier dispositif et du deuxième dispositif est en outre configurée pour sélectionner une ou plusieurs d'une pluralité d'électrodes sous-crâniennes à activer.

4. Système selon l'une quelconque des revendications 1 à 3, dans lequel le premier dispositif et le deuxième dispositif comprennent chacun en outre une antenne, chaque antenne pouvant recevoir des données en provenance d'un programmateur externe ou transmettre des données à un programmateur externe, optionnellement dans lequel la circuiterie de commande du premier dispositif et du deuxième dispositif est en outre configurée pour programmer des paramètres de traitement de chaque premier dispositif ou deuxième dispositif respectif en fonction des commandes en provenance du programmateur externe, les paramètres de traitement étant sélectionnés dans un groupe constitué de la sélection des électrodes actives, de la fréquence d'impulsion, de l'amplitude d'impulsion, de la largeur d'impulsion, du cycle de service, de l'initiation du traitement et de l'arrêt du traitement.

5. Système selon la revendication 1, dans lequel la circuiterie de commande de chaque premier dispositif et deuxième dispositif respectif comprend en outre un amplificateur d'EEG et un convertisseur analogique-numérique, permettant ainsi au premier dispositif et au deuxième dispositif d'enregistrer un EEG du patient.

6. Système selon l'une quelconque des revendications 1 à 5, dans lequel chacun du premier dispositif et du deuxième dispositif comprend en outre une mémoire configurée pour le stockage de l'EEG de la personne (403), et une antenne émettrice, la circuiterie de commande de chaque premier dispositif et deuxième dispositif respectif étant configurée pour transmettre l'EEG stocké par l'intermédiaire de son antenne émettrice respective.

7. Système selon l'une quelconque des revendications 1 à 6, comprenant en outre un interrupteur magnétique, dans lequel le démarrage ou l'arrêt d'un enregistrement d'EEG est commandé par le fait que la personne place un aimant permanent à proximité de l'interrupteur magnétique, ou que la personne éloigne l'aimant permanent de l'interrupteur magnétique.

8. Système selon l'une quelconque des revendications 1 à 6, dans lequel chacun du premier dispositif et du deuxième dispositif comprend en outre un interrupteur magnétique, le démarrage ou l'arrêt de la stimulation étant commandé par le fait que la personne place un aimant permanent à proximité de l'interrupteur magnétique, ou que la personne éloigne l'aimant permanent de l'interrupteur magnétique.

9. Système selon l'une quelconque des revendications 1 à 8, dans lequel le fil de chaque premier dispositif et deuxième dispositif respectif est configuré pour être implanté de telle sorte qu'une ou plusieurs des électrodes sous-crâniennes se trouvent dans ou près d'un noyau antérieur d'un thalamus, dans ou près d'un noyau accumbens, dans ou près d'un noyau sous-thalamique ou d'un globus pallidus interne, dans ou près d'un hippocampe, dans ou près d'un gyrus cingulaire sous-calleux, dans ou près d'un noyau antérieur d'une glande pituitaire, dans ou près d'une formation réticulaire du mésencéphale ou d'un noyau thalamique non spécifique, ou dans ou près d'un cortex du patient.

10. Système selon la revendication 1, comprenant en outre un fil conducteur configuré pour connecter les unes aux autres les électrodes sous-cutanées du premier dispositif et du deuxième dispositif.

11. Système selon la revendication 1, dans lequel le premier dispositif comprend en outre un haut-parleur configuré pour fournir des informations audio à la personne, à un clinicien ou à un soignant.

12. Système selon la revendication 11, dans lequel les informations audio sont générées sous la forme d'une série de bips, de tics ou de tonalités, qui peuvent être décodés.
